# EUROPEAN PATENT SPECIFICATION

(11) **EP 1 408 047 B1**
(45) Date of publication and mention of the grant of the patent: **26.10.2005**
(21) Application number: 02738658.0
(22) Date of filing: 12.06.2002
(51) Int. Cl.: C07H 17/08

(54) **PROCESS FOR PREPARATION OF ERYTHROMYCIN COMPOUNDS**
VERFAHREN ZUR HERSTELLUNG VON ERYTHROMYCINVERBINDUNGEN
PROCEDE DE PREPARATION DE COMPOSES DE TYPE ERYTHROMYCINE

(30) Priority: 13.06.2001 JP 2001178001
(43) Date of publication of application: 14.04.2004
(73) Proprietor: Ube Industries, Ltd., Ube-Shi, Yamaguchi 755-8633 (JP); CHUGAI SEIYAKU KABUSHIKI KAISHA, Tokyo, 115-8543 (JP)
(72) Inventor: MIYATA, Hiroyuki Ube Res. Lab., Ube Ind., Ltd., Ube-shi, Yamaguchi 755-8633 (JP); Takama, Akira Ube Res. Lab., Ube Ind., Ltd, Ube-shi, Yamaguchi 755-8633 (JP); Yamamoto, Yasuhito Ube Res. Lab., Ube Ind., Ltd., Ube-shi, Yamaguchi 755-8633 (JP); ATAKA, Kikuo Ube Res. Lab., Ube Ind., Ltd., Ube-shi, Yamaguchi 755-8633 (JP)
(74) Representative: Henkel, Feiler & Hänzel
(86) International application number: PCT/JP2002/005825
(87) International publication number: WO 2002/102818

(56) References cited:
- EP-A1- 0 643 068
- EP-A1- 0 846 697
- WO-A1-94/10185

## Description

### Technical field

The present invention relates to a process for preparing an erythromycin compound which contains a method of alkylating an erythromycin compound.

### Background art

Compound A (11-oxo-12-alkoxy-8,9-anhydroerythromycin A 6,9-hemiketal) which is an erythromycin compound represented by the formula: 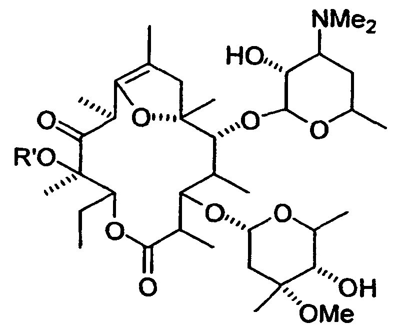 wherein R' represents a lower alkyl group, is a compound useful as a synthetic raw material of a gastro-prokinetic agent (for example, see de(N-methyl)-11-deoxy-N-isopropyl-12-O-methyl-11-oxo-8,9-anhydroxyerythromycin A 6,9-hemiacetal (GM-611), New Current 7(13), pp. 19-21, issued on June 10, 1996). A method for producing the erythromycin compound has been already known. Of these, a method of producing Compound A from Compound B (2'-O-acetyl-4"-O-formyl-11-oxo-8,9-anhydroerythromycin A 6,9-hemiketal) represented by the formula: 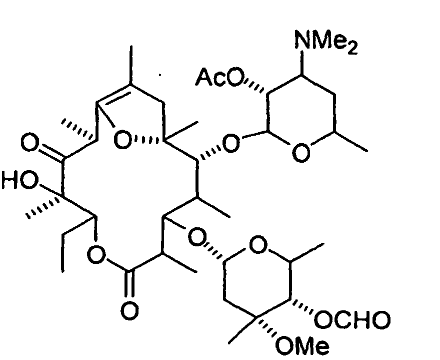 through Compound C (2'-O-acetyl-4"-O-formyl-11-oxo-12-alkoxy-8,9-anhydroerythromycin A 6,9-hemiketal) represented by the formula: 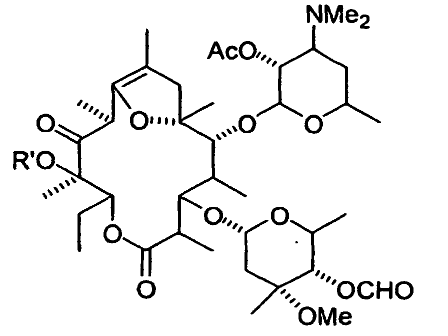 wherein R' has the same meaning as defined above, has been disclosed in Japanese Provisional Patent Publication No. 100291/1997 (which corresponds to U.S. Patent No. 5,959,088). This method is to react Compound B with methyl tosylate in the presence of sodium hydride in an aprotic polar solvent (dimethylformamide in Example) to form Compound C, and then, deprotecting the resulting compound to obtain Compound A with a yield of 59% (based on Compound B). However, in this method, there are problems that a yield of Compound A is low, and the resulting crystal is colored (that is, a purity is low and a quality is bad), and further the operation is troublesome and the like, so that it is unpractical as an industrial preparation process.

EP-A-0 643 068 discloses a compound represented by general formula [I] or a salt thereof, being extremely reduced in the decomposability by gastric juice as compared with other known erythromycin derivatives and having an excellent activity of promoting the movement of digestive tracts, wherein R₁ represents hydrogen or acyl; R₂ and R₃ may be the same or different from each other and each represents hydrogen, hydroxyl, acyloxy or amino, or alternatively R₂ and R₃ are combined together to represent =O or =NOR₁₀, wherein R₁₀ represents hydrogen or lower alkyl; R₄ represents hydrogen or lower alkyl; and Y represents -NR₅R₆ or -N⁺R-₇R₈R₉X⁻, wherein R₅, R₆, R₇, R₈ and R₉ may be the same or different from one another and each represents hydrogen, optionally substituted lower alkyl, lower alkenyl, lower alkynyl or cycloalkyl, or a 3- to 7-membered heterocyclic group containing oxygen, nitrogen or sulfur as the heteroatom, and X represents an anion, provided that a pair of R₅ and R₆ and a pair of R₇ and R₈ may be each combined with the adjacent nitrogen atom to represent azacycloalkyl.

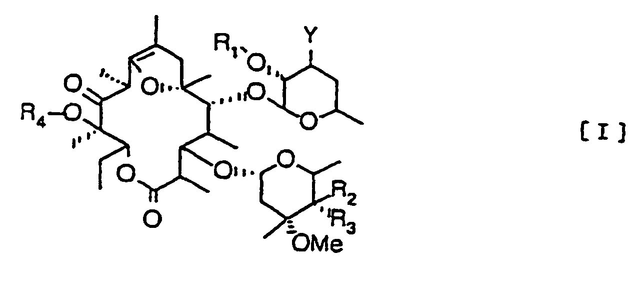

To solve the above mentioned problems, the present inventors have carried out earnest studies to seek a method which can obtain a 11-oxo-12-alkoxy-8,9-anhydroerythromycin A 6,9-hemiketal compound such as Compound A with a simple and easy method and a high yield and high purity by subjecting a 2'-O-acetyl-4"-O-formyl-11-oxo-8,9-anhydroerythromycin A 6,9-hemiketal compound such as Compound B to alkylation, and then, to deprotection. As a result , they have found that the alkylation proceeds with a high yield and high purity when alkylation of the 2'-O-acetyl-4"-O-formyl-11-oxo-8,9-anhydroerythromycin A 6,9-hemiketal compound is carried out in the presence of a base in a reaction system in which a specific amount of water is contained to an amount of the compound, whereby they have completed the present invention.

### Disclosure of the invention

The present invention relates to a process for alkylating 2'-O-acetyl-4"-O-formyl-11-oxo-8,9-anhydroerythromycin A 6,9-hemiketal compound (Compound 1) represented by the formula (1): 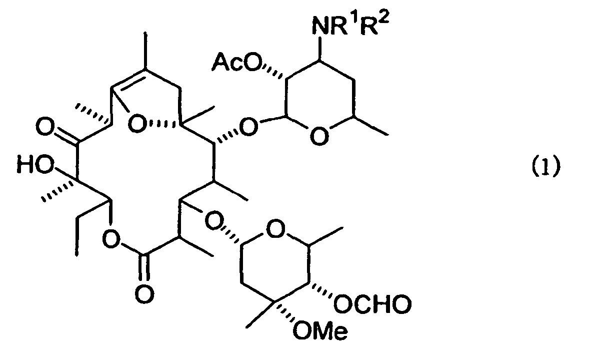 wherein R¹ and R² each independently represent a lower alkyl group,
which comprises reacting Compound 1 with an alkylating agent in the presence of a base in a mixed solvent of 0.18 to 1.05 equivalent of water based on the amount of Compound 1 and an organic solvent, to obtain 2'-O-acetyl-4"-O-formyl-11-oxo-12-alkoxy-8,9-anhydroerythromycin A 6,9-hemiketal compound (Compound 3) represented by the formula (3): 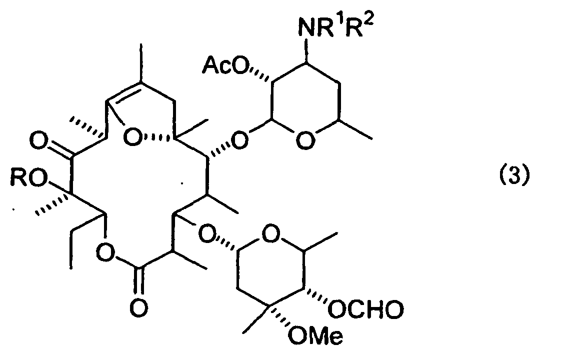 wherein R represents a lower alkyl group, R¹ and R² have the same meanings as defined above.

The present invention also relates to a process for preparing a 11-oxo-12-alkoxy-8,9-anhydroerythromycin A 6,9-hemiketal compound (Compound 2) represented by the formula (2) : 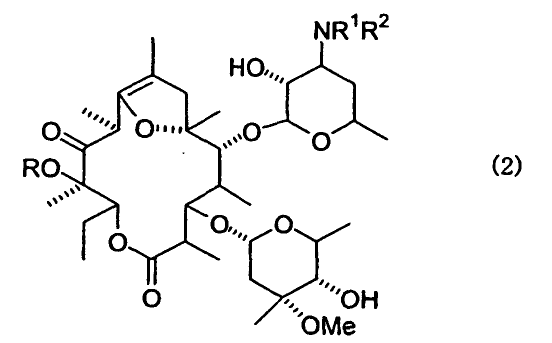 wherein R, R¹ and R² have the same meanings as defined above,
by further removing the acetyl group at 2'-position and the formyl group at 4"-position of the obtained Compound 3 after the above-mentioned alkylation.

### Best mode for carrying out the invention

The above-mentioned processes of the present invention are shown by the following reaction scheme. 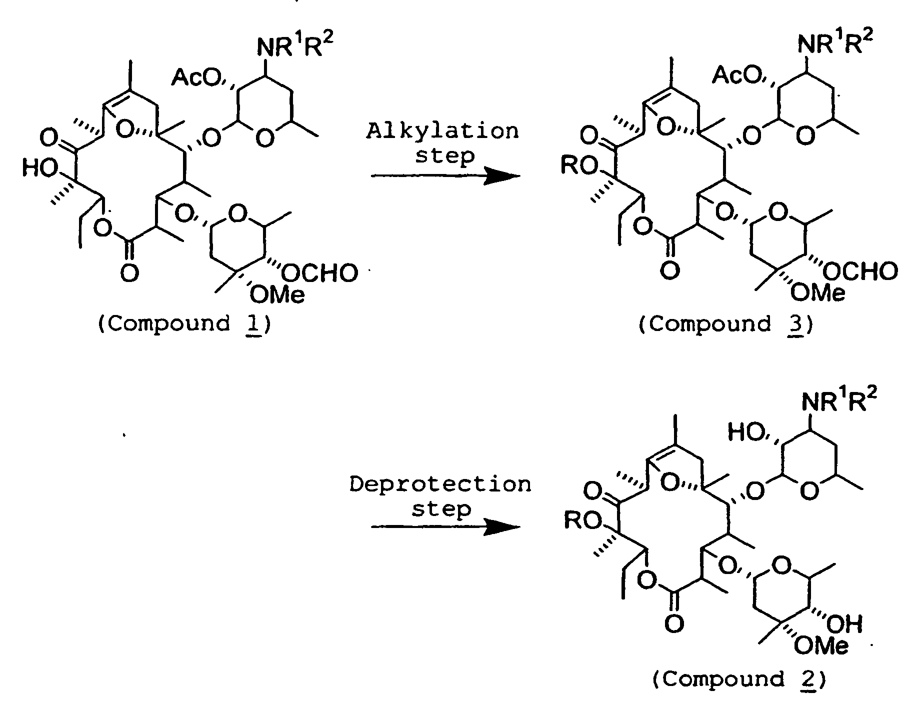 wherein R, R¹ and R² have the same meanings as defined above.

In Compound 1 which can be alkylated according to the process of the present invention, R¹ and R² are each independently a lower alkyl group, and as the lower alkyl group, there may be specifically mentioned a straight or branched alkyl group having 1 to 4 carbon atoms including, for example, a methyl group, an ethyl group, a n-propyl group, an isopropyl group, a n-butyl group, an isobutyl group, a sec-butyl group, a tert-butyl group and the like. There may be preferably a methyl group, an ethyl group, a n-propyl group and isopropyl group. Of these, preferred is a case where R¹ and R² are both methyl groups, and a case wherein R¹ is a methyl group and R² is an isopropyl group.

### (A) Alkylation step

In the method of the present invention, alkylation can be carried out, in the presence of a base, Compound 1 is reacted with an alkylating agent having an alkyl group to be desired to be introduced in a mixed solvent of water in an amount of about 0.18 to 1.05 equivalent based on the amount of Compound 1 and an organic solvent. By carrying out the alkylation in the presence of water in a total amount of about 0.18 to 1.05 equivalent based on the amount of Compound 1 in the reaction system before the reaction, Compound 3 which is a compound in which a hydroxyl group at 12-position of Compound 1 is alkylated by a desired alkyl group can be obtained.

In this alkylation step, alkylation can be carried out by an alkylating agent having a desired lower alkyl group depending on the use. Of these, the alkylation can be specifically carried out by using an alkylating agent having a straight or branched alkyl group with 1 to 6 carbon atoms, for example, a methyl group, an ethyl group, a n-propyl group, an isopropyl group, a n-butyl group, an isobutyl group, a sec-butyl group, a tert-butyl group, a pentyl group, a hexyl group, and the alkylation can be carried out by an alkylating agent preferably having a methyl group, an ethyl group, a n-propyl group or an isopropyl group, more preferably a methyl group.

In the step the alkylation, various kinds of alkylating agents having the above-mentioned desired alkyl group may be used, and there may be used, for example, an alkyl halide such as methyl iodide, ethyl iodide, etc.; a dialkylsulfate such as dimethyl sulfate, diethylsulfate, etc.; an alkylsulfonic acid alkyl ester such as methyl methanesulfonate, methyl ethanesulfonate, ethyl methanesulfonate, etc.; and an arylsulfonic acid alkyl ester such as methyl benzenesulfonate, methyl p-toluenesulfonate, methyl p-bromobenzenesulfonate, ethyl p-toluenesulfonate, etc. It is preferably used a dialkylsulfate, an alkylsulfonic acid alkyl ester, an arylsulfonic acid alkyl ester, more preferably a dialkylsulfate and an arylsulfonic acid alkyl ester, particularly preferably a dialkylsulfate. For example, methylation is to be carried out, dimethyl sulfate and methyl p-toluenesulfonate, particularly preferably dimethyl sulfate can be used.

An amount of the above-mentioned alkylating agent is preferably about 1.0 to 5.0-fold mol, more preferably about 1.0 to 1.2-fold mol based on the amount of Compound 1.

In the step of the alkylation, a total amount of water to be present in the reaction system before the reaction is about 0.18 to 1.05 equivalent, preferably about 0.18 to 0.80 equivalent, more preferably about 0.18 to 0.70 equivalent based on the amount of Compound 1. If the total amount of water to be present in the reaction system is out of the above-mentioned range, a yield after the alkylation is low.

As the base to be used in the step of the alkylation, there may be mentioned, for example, an alkali metal or alkaline earth metal hydride such as sodium hydride, potassium hydride, calcium hydride, etc.; an alkali metal hydroxide such as sodium hydroxide, potassium hydroxide, etc.; and an alkali metal alkoxide such as sodium methoxide, sodium ethoxide, potassium tert-butoxide, etc., preferably alkali metal halide, more preferably sodium hydride can be used. These bases may be used singly or in combination of two or more kinds in admixture.

An amount of the above-mentioned base to be used is preferably about 1.0 to 5.0-fold mol, more preferably about 1.0 to 1.5-fold mol based on the amount of Compound 1.

As the organic solvent to be used in the step of the alkylation, it is not specifically limited so long as it does not inhibit the reaction, and there may be preferably mentioned an ether, and, for example, diethyl ether, diisopropyl ether, dibutyl ether, tetrahydrofuran, 1,4-dioxane, etc. may be used, more preferably tetrahydrofuran can be used. These organic solvents may be used singly or in combination of two or more kinds in admixture.

An amount of the above-mentioned organic solvent to be used may be optionally controlled depending on uniformity of the reaction solution and stirrability of the same, and it is preferably used in an amount about 100 to 10,000 ml, more preferably about 500 to 5,000 ml based on 1 mol of Compound 1.

The step of the alkylation can be carried out, for example, in the atmosphere of an inert gas (for example, nitrogen, argon, helium, etc.), by mixing a base, Compound 1, about 0.18 to 1.05 equivalent of water based on the amount of Compound 1, an organic solvent and an alkylating agent to carry out the reaction, and the like. It is preferably carry out the reaction by once mixing a base, Compound 1, about 0.18 to 1.05 equivalent of water based on the amount of Compound 1 and an organic solvent, and then, adding an alkylating agent to the mixture to carry out the reaction. A reaction temperature at that time is preferably about -20 to 70°C, more preferably about -10 to 20°C, and a reaction pressure is not specifically limited. A reaction time is about 2 to 24 hours.

Compound 3 obtained by the step of the alkylation can be isolated and purified after completion of the reaction according to the conventionally known method such as distillation, recrystallization, column chromatography, etc. However, when removals of the acetyl group and the formyl group are carried out successively, alkylation and removals of the acetyl group and the formyl group of Compound 3 are preferably carried out by a one-pot method wherein isolation and purification of Compound 3 after the alkylation are not carried out in view of the operation of the reaction.

### (B) Deprotection step for removing acetyl group and formyl group

After the alkylation according to the method of the present invention, deprotection in which the acetyl group at the 2'-position and the formyl group at the 4"-position of the obtained Compound 3 are removed is carried out to obtain Compound 2.

The step of the deprotection is not specifically limited so long as it is a general removal method of the acetyl group and the formyl group, and it is preferably carried out in basic conditions in a solvent.

As a base to be used for making the reaction system basic in the step of the deprotection, there may be mentioned, for example, sodium carbonate, sodium hydrogen carbonate, potassium carbonate, potassium hydrogen carbonate, sodium hydroxide, etc., preferably sodium hydrogen carbonate can be used. These bases may be used singly or in combination of two or more kinds in admixture.

An amount of the above-mentioned base to be used can be optionally controlled depending on the stirrability of the reaction solution or stirrability of the same, and it is preferably used in an amount about 0.1 to 20-fold mol, more preferably about 0.5 to 20-fold mol, particularly preferably about 0.5 to 5-fold mol based on the amount of Compound 1 used in the step of the alkylation.

As the solvent to be used in the deprotection step, it is not specifically limited so long as it does not interfere the reaction, and preferably mentioned a lower alcohol, or a mixture of these lower alcohols and water, and, for example, methanol, ethanol, or a mixed solvent of these with water may be used.

An amount of the above-mentioned solvent is preferably about 100 to 10000 ml, more preferably about 500 to 5000 ml based on 1 mol of Compound 1 used in the alkylation step. Incidentally, when a mixed solvent of a lower alcohol and water is used, a mixing ratio is preferably about 0.1 to 5-fold volume, more preferably about 0.5 to 3-fold volume based on the amount of a lower alcohol.

The deprotection step may be carried out, for example, in an inert gas (for example, nitrogen, argon, helium, etc.) atmosphere, Compound 3 (which may not be isolated or purified), a base and a solvent are mixed and reacted to each other, and the like. A reaction temperature at that time is preferably about 0 to 100°C, more preferably about 40 to 80°C, particularly preferably about 50 to 60°C, and a reaction pressure is not specifically limited. Also, a reaction time is about 3 to 20 hours.

Compound 2 obtained by the above-mentioned deprotection step can be isolated and purified after completion of the reaction, according to the conventional method such as distillation, recrystallization, column chromatography, etc., and by carrying out the following purification step successively, Compound 2 with a higher purity can be obtained.

### (C) Purification step

This purification is carried out by heating Compound 2 obtained in the above-mentioned deprotection step under stirring in a saturated hydrocarbon solvent, whereby crystals of Compound 2 with higher purity can be obtained.

As a saturated hydrocarbon solvent to be used in the purification step, there may be preferably mentioned a straight, branched or cyclic saturated hydrocarbons having 5 to 12 carbon atoms, more specifically, for example, pentane, hexane, heptane, octane, nonane, decane (various kinds of isomers are included in the above-mentioned hydrocarbons), cyclopentane, cyclohexane, cyclopentane, etc. can be used. It is preferably used hexane and/or cyclohexane, more preferably hexane. These saturated hydrocarbon solvents may be used singly or in combination of two or more in admixture.

An amount of the above-mentioned saturated hydrocarbon solvent to be used is preferably about 100 to 10000 ml, more preferably about 500 to 5000 ml based on 1 mol of Compound 1 used in the alkylation step.

The purification step can be carried out, for example, by the method in which Compound 2 obtained by the above-mentioned deprotection step and a saturated hydrocarbon solvent are mixed, heated preferably about 35 to 100°C, more preferably about 50 to 80°C, and stirred for about 0.5 to 10 hours, etc. Incidentally, purified crystals of Compound 2 with higher purity can be easily isolated by drying after filtration.

Incidentally, Compound 1 represented by the formula (1) which is a starting compound of the process according to the present invention can be prepared according to the method as disclosed in Japanese Provisional Patent Publication No. Hei. 9-100291 (which corresponds to U.S. Patent No. 5,959,088), and by a process well known in this field of the art.

### Example

Next, the present invention is specifically explained by referring to Examples and Comparative examples, but the scope of the present invention is not limited by these.

### Example 1

To a flask having an inner volume of 300 ml equipped with a stirring device, a thermometer and a dropping funnel were charged, under nitrogen atmosphere, 20.0 g (25.5 mmol) of 2'-O-acetyl-4"-O-formyl-11-oxo-8,9-anhydroerythromycin A 6,9-hemiketal (Compound 1, obtained according to the method disclosed in Japanese Provisional Patent Publication No. 100291/1997) and 80 ml of tetrahydrofuran, and the mixture was made uniform by stirring at room temperature. When the water content in the system of this solution was measured, it was 1582 ppm (Analytical value by Karl Fischer's water content measurement device). 56 µl (3.11 mmol) of water was added to the solution. The total water content in the system at this time was 0.43 equivalent based on the amount of Compound 1. Then, 1.33 g (33.2 mmol) of 60% sodium hydride and 2.57 ml (27.2 mmol) of dimethyl sulfate were gradually added dropwise to the mixture in this order in an ice-bath and maintaining to 5°C, to carry out an alkylation reaction at 5 to 10°C for 4 hours. Thereafter, 102 mg (2.55 mmol) of 60% sodium hydride and 0.048 ml (0.51 mmol) of dimethyl sulfate were further added to the mixture to carry out the alkylation reaction for 2 hours. After completion of the alkylation reaction, 200 ml of an ice-cold saturated aqueous ammonium chloride solution was added to the mixture and the resulting mixture was stirred for 15 minutes, and then, 330 ml of ethyl acetate was added to the same. Then, insoluble materials were filtered off, the ethyl acetate layer was collected by separation, washed twice with each 200 ml of water, and dried over anhydrous magnesium sulfate. After filtration, the filtrate was concentrated under reduced pressure, and further, 200 ml of hexane was added to the concentrate to precipitate crystals. The obtained crystals were filtered and dried to obtain 17.3 g of 2'-O-acetyl-4"-O-formyl-11-oxo-12-methoxy-8,9-anhydroerythromycin A 6,9-hemiketal (Compound 3) (Isolated yield based on Compound 1: 85%) as white crystals with a purity of 90% (analytical value by high performance liquid chromatography, analytical condition 1).

Physical properties of Compound 3 were as mentioned below.
Melting point: 229 to 235°C
FAB-MS; 798 (MH+)
¹H-NMR (CDCl₃, δ (ppm)); 0.95 (3H, t), 2.04 (3H, s), 2.26 (6H, s), 3.05 (3H, s), 3.35 (3H, s), 4.52 (1H, d), 5.02 (1H, d), 5.63 (1H, dd), 8.19 (1H, d)

### Example 2

To a flask having an inner volume of 200 ml equipped with a stirring device, a thermometer and a reflux condenser were charged 12.0 g (15 mmol) of 2'-O-acetyl-4"-O-formyl-11-oxo-12-methoxy-8,9-anhydroerythromycin A 6,9-hemiketal (Compound 3) obtained in Example 1, 24 ml (27 mmol) of a saturated aqueous sodium hydrogen carbonate solution and 46 ml of methanol, and a deprotection reaction was carried out under reflux (60 to 65°C) for 3.5 hours under nitrogen gas atmosphere. After completion of the deprotection reaction, the mixture was concentrated under reduced pressure, and 120 ml of ethyl acetate and 30 ml of water were added to the concentrate. The ethyl acetate layer was collected by separation, washed twice with each 60 ml of water and dried over anhydrous magnesium sulfate. After filtration, the mixture was concentrated under reduced pressure, 19 ml of acetone and 15 ml of 10% aqueous ammonia were added to the concentrate, and the mixture was stirred at room temperature for one hour, and at 0 to 5°C for one hour to precipitate crystals. The obtained crystals were filtered and dried to give 9.9 g of 11-oxo-12-methoxy-8,9-anhydroerythromycin A 6,9-hemiketal (Compound 2) (Isolated yield based on Compound 3: 90%) as white crystals with a purity of 98% (analytical value by high performance liquid chromatography, analytical condition 2).

Physical properties of Compound 2 were as mentioned below.
Melting point: 197 to 199°C
¹H-NMR(CDCl₃, δ (ppm)); 0.93 (3H, t), 1.30 (3H, s), 1.90 to 2.10 (1H, dd), 2.34 (6H, s), 2.40 to 2.70 (4H, m), 3.01 to 3.08 (4H, m), 3.22 (1H, dd), 3.32 (3H, s), 3.50 to 4.10 (5H, m), 4.36 (1H, d), 4.95 (1H, d), 5.60 (1H, d)

### Example 3

To a flask having an inner volume of 500 ml equipped with a stirring device, a thermometer, a reflux condenser and a dropping funnel were charged 225 µl (12.5 mmol) of water, 110 ml of tetrahydrofuran and 2.98 g (74 mmol) of 60% sodium hydride under nitrogen atmosphere, and the mixture was stirred at 17°C for 20 minutes. Then, 45.0 g (57.4 mmol) of 2'-O-acetyl-4"-O-formyl-11-oxo-8,9-anhydroerythromycin A 6,9-hemiketal (Compound 1) and 25 ml of tetrahydrofuran were added to the mixture, and the resulting mixture was stirred at the same temperature for 20 minutes. The total water content in the system at this time was 0.30 equivalent relative to Compound 1. Thereafter, 5.70 ml (60.3 mmol) of dimethyl sulfate was gradually added to the mixture to carry out an alkylation reaction at the same temperature for 2 hours.

After completion of the alkylation reaction, 93 ml (103 mmol) of a saturated aqueous sodium hydrogen carbonate solution and 176 ml of methanol were added to the mixture to carry out a deprotection reaction under reflux (65°C) for 7 hours under nitrogen gas atmosphere. After completion of the deprotection reaction, the mixture was concentrated under reduced pressure, and 455 ml of ethyl acetate and 220 ml of water were added to the mixture. The ethyl acetate layer was collected by separation, washed twice with each 220 ml of water and dried over anhydrous magnesium sulfate. After filtration, the mixture was concentrated under reduced pressure, 73.1 ml of acetone and 56.3 ml of 10% aqueous ammonia were added to the concentrate, and the resulting mixture was stirred at room temperature for one hour, and at 0 to 5°C for one hour to precipitate crystals. The obtained crystals were filtered and dried to give 33.7 g of 11-oxo-12-methoxy-8,9-anhydroerythromycin A 6,9-hemiketal (Compound 2) (Isolated yield based on Compound 1: 81%) as white crystals with a purity of 96% (analytical value by high performance liquid chromatography, analytical condition 2).

Subsequently, the above-mentioned Compound 2 with a purity of 96% and 160 ml of n-hexane were mixed, and the mixture was stirred under reflux (67°C) for 30 minutes, and further at 5 to 15°C for 30 minutes. The obtained crystals were filtered and dried to give 29.3 g of Compound 2 (Recovery rate: 91%) as white crystals with a purity of 98% (analytical value by high performance liquid chromatography, analytical condition 2).

Physical property of Compound 2 was as follows.
Melting point:197 to 199°C

### Example 4

To a flask having an inner volume of 200 ml equipped with a stirring device, a thermometer, a reflux condenser and a dropping funnel were charged 149 µl (8.27 mmol) of water, 70 ml of tetrahydrofuran and 1.33 g (33.5 mmol) of 60% sodium hydride under nitrogen atmosphere, and the mixture was stirred at 17°C for 10 minutes. Then, 20.0 g (25.5 mmol) of 2'-O-acetyl-4"-O-formyl-11-oxo-8,9-anhydroerythromycin A 6,9-hemiketal (Compound 1) and 10 ml of tetrahydrofuran were added to the mixture, and the resulting mixture was stirred at the same temperature for 10 minutes. The total water content in the system at this time was 0.66 equivalent relative to Compound 1. Thereafter, 2.53 ml (26.7 mmol) of dimethyl sulfate was gradually added dropwise to the mixture to carry out an alkylation reaction at the same temperature for 2.5 hours. Moreover, 0.048 ml (0.51 mmol) of dimethyl sulfate was further added to the mixture to carry out the alkylation reaction for 1.25 hours, 132 mg (2.6 mmol) of 60% sodium hydride and 0.048 ml (0.51 mmol) of dimethyl sulfate were further added to the same for 1.5 hours, 0.048 ml (0.51 mmol) of dimethyl sulfate was further added to the same for 1.5 hours, and 132 mg (2.6 mmol) of 60% sodium hydride and 0.048 ml (0.51 mmol) of dimethyl sulfate were further added to the same for 1.5 hours.

After completion of the alkylation reaction, 41 ml (45 mmol) of a saturated aqueous sodium hydrogen carbonate solution and 78 ml of methanol were added to the mixture to carry out a deprotection reaction under reflux (65°C) for 7 hours under nitrogen gas atmosphere. After completion of the deprotection reaction, the mixture was concentrated under reduced pressure, and 200 ml of ethyl acetate and 100 ml of water were added to the concentrate. The ethyl acetate layer was collected by separation, washed twice with each 100 ml of water and dried over anhydrous magnesium sulfate. After filtration, the mixture was concentrated under reduced pressure, 32.5 ml of acetone and 25.0 ml of 10% aqueous ammonia were added to the concentrate, and the mixture was stirred at room temperature for one hour, and at 0 to 5°C for one hour to precipitate crystals. The obtained crystals were filtered and dried to give 14.5 g of 11-oxo-12-methoxy-8,9-anhydroerythromycin A 6,9-hemiketal (Compound 2) (Isolated yield based on Compound 1: 78%) as white crystals with a purity of 96% (analytical value by high performance liquid chromatography, analytical condition 2).

Subsequently, the above-mentioned Compound 2 with a purity of 96% and 70 ml of n-hexane were mixed, and the mixture was stirred under reflux (67°C) for 30 minutes, and at 5 to 15°C for 30 minutes. The obtained crystals were filtered and dried to give 12.0 g of Compound 2 (Recovery rate: 86%) as white crystals with a purity of 98% (analytical value by high performance liquid chromatography, analytical condition 2).

### Example 5

To a flask having an inner volume of 100 ml equipped with a stirring device, a thermometer, a reflux condenser and a dropping funnel were charged 20 ml of tetrahydrofuran and 730 mg (18.3 mmol) of 60% sodium hydride under nitrogen atmosphere, and the mixture was stirred at 29°C for 20 minutes. Then, 11.0 g (14.0 mmol) of 2'-O-acetyl-4"-O-formyl-11-oxo-8,9-anhydroerythromycin A 6,9-hemiketal (Compound 1) and 13 ml of tetrahydrofuran were added to the mixture, and the resulting mixture was stirred at the same temperature for 10 minutes. The total water content in the system at this time was 0.37 equivalent relative to Compound 1. Thereafter, 1.39 ml (14.7 mmol) of dimethyl sulfate was gradually added dropwise to the mixture to carry out an alkylation reaction at the same temperature for 2 hours.

After completion of the alkylation reaction, 23 ml (25 mmol) of a saturated aqueous sodium hydrogen carbonate solution and 43.3 ml of methanol were added to the reaction mixture to carry out a deprotection reaction under reflux (65°C) for 8 hours under nitrogen gas atmosphere. After completion of the deprotection reaction, the mixture was concentrated under reduced pressure, and 111 ml of ethyl acetate and 30 ml of water were added to the concentrate. The ethyl acetate layer was collected by separation, washed twice with each 30 ml of water and dried over anhydrous magnesium sulfate. After filtration, the mixture was concentrated under reduced pressure, 17.9 ml of acetone and 13.8 ml of 10% aqueous ammonia were added to the concentrate, and the resulting mixture was stirred at room temperature for one hour, and at 0 to 5°C for one hour to precipitate crystals. The obtained crystals were filtered and dried to give 7.97 g of 11-oxo-12-methoxy-8,9-anhydroerythromycin A 6,9-hemiketal (Compound 2) (Isolated yield based on Compound 1: 78%) as white crystals with a purity of 97% (analytical value by high performance liquid chromatography, analytical condition 2).

### Example 6

To a flask having an inner volume of 100 ml equipped with a stirring device, a thermometer, a reflux condenser and a dropping funnel were charged 8.03 g (10.24 mmol) of 2'-O-acetyl-4"-O-formyl-11-oxo-8,9-anhydroerythromycin A 6,9-hemiketal (Compound 1) and 32 ml of tetrahydrofuran under nitrogen atmosphere, and the mixture was stirred at room temperature to make uniform. When the water content in the system of this solution was measured, it was 1330 ppm (Analytical value by Karl Fischer's water content measurement device). To the solution was added 8 µl (0.44 mmol) of water. Then, 532 mg (13.31 mmol) of 60% sodium hydride was added to the solution while maintaining a liquid temperature to 5°C in an ice-bath, and after stirring for 5 minutes, 24.1 µl (1.34 mmol) of water was added to the solution and the solution was stirred for 10 minutes. The total water content in the system at this time was 0.43 equivalent relative to Compound 1. Then, 1.02 ml (10.75 mmol) of dimethyl sulfate was gradually added dropwise to the solution to carry out an alkylation reaction at 5 to 10°C for 5 hours, and 40.8 mg (1.02 mmol) of 60% sodium hydride and 19.4 µl (0.20 mmol) of dimethyl sulfate were further added to the solution to carry out the alkylation reaction for one hour.

After completion of the alkylation reaction, 17 ml (19 mmol) of a saturated aqueous sodium hydrogen carbonate solution and 31 ml of methanol were added to the mixture to carry out a deprotection reaction under reflux (60 to 65°C) for 7 hours under nitrogen gas atmosphere. After completion of the deprotection reaction, the mixture was concentrated under reduced pressure, and 81 ml of ethyl acetate and 40 ml of water were added to the reaction mixture. The ethyl acetate layer was collected by separation, washed twice with each 40 ml of water and dried over anhydrous magnesium sulfate. After filtration, the mixture was concentrated under reduced pressure, 13 ml of acetone and 10 ml of 10% aqueous ammonia were added to the concentrate, and the mixture was stirred at room temperature for one hour, and at 0 to 5°C for one hour to precipitate crystals. The obtained crystals were filtered and dried to give 6.82 g of 11-oxo-12-methoxy-8,9-anhydroerythromycin A 6,9-hemiketal (Compound 2) (Isolated yield based on Compound 1: 92%) as white crystals with a purity of 96% (analytical value by high performance liquid chromatography, analytical condition 2).

### Example 7

To a flask having an inner volume of 5000 ml equipped with a stirring device, a thermometer, a reflux condenser and a dropping funnel were charged 400 g (0.51 mol) of 2'-O-acetyl-4"-O-formyl-11-oxo-8,9-anhydroerythromycin A 6,9-hemiketal (Compound 1) and 1600 ml of tetrahydrofuran under nitrogen atmosphere, and the mixture was stirred at room temperature to make uniform. When the water content in the system of this solution was measured, it was 2133 ppm (Analytical value by Karl Fischer's water content measurement device). To the solution was added 0.11 ml (6.1 mmol) of water at room temperature, and 26.5 g (633 mmol) of 60% sodium hydride was added to the solution while maintaining the liquid temperature to 0 to 10°C in an ice-bath and the resulting mixture was stirred for 5 minutes. The total water content in the system at this time was 0.43 equivalent relative to Compound 1. Then, 51 ml (0.54 mol) of dimethyl sulfate was gradually added dropwise to the solution to carry out an alkylation reaction at 5 to 10°C for 2 hours.

After completion of the alkylation reaction, 823 ml (909 mmol) of a saturated aqueous sodium hydrogen carbonate solution and 1560 ml of methanol were added to the solution to carry out a deprotection reaction under reflux (60 to 65°C) for 7 hours under nitrogen gas atmosphere. After completion of the deprotection reaction, the mixture was concentrated under reduced pressure, and 4050 ml of ethyl acetate and 2000 ml of water were added to the concentrate. The ethyl acetate layer was collected by separation, washed twice with each 2000 ml of water and dried over anhydrous magnesium sulfate. After filtration, the mixture was concentrated under reduced pressure, 650 ml of acetone and 500 ml of 10% aqueous ammonia were added to the concentrate, and the mixture was stirred at room temperature for one hour, and at 0 to 5°C for one hour to precipitate crystals. The obtained crystals were filtered and dried to give 315.9 g of 11-oxo-12-methoxy-8,9-anhydroerythromycin A 6,9-hemiketal (Compound 2) (Isolated yield based on Compound 1: 87%) as white crystal with a purity of 98% (analytical value by high performance liquid chromatography, analytical condition 2).

Subsequently, the above-mentioned Compound 2 with a purity of 98% and 1580 ml of n-hexane were mixed, and the mixture was stirred under reflux (65 to 70°C) for 30 minutes, and at 5 to 15°C for 30 minutes. The obtained crystals were filtered and washed with 316 ml of n-hexane, and then, dried to give 293.8 g of Compound 2 (Recovery rate: 93%) as white crystals with a purity of 98% (analytical value by high performance liquid chromatography, analytical condition 2).

### Example 8

To a flask having an inner volume of 100 ml equipped with a stirring device, a thermometer, a reflux condenser and a dropping funnel were charged 10.0 g (12.76 mmol) of 2'-O-acetyl-4"-O-formyl-11-oxo-8,9-anhydroerythromycin A 6,9-hemiketal (Compound 1) and 90 ml of tetrahydrofuran under nitrogen atmosphere, the mixture was stirred at room temperature to make it uniform, and then, 50 ml of tetrahydrofuran was removed by distillation. When the water content in the system of this solution was measured, it was 313 ppm (Analytical value by Karl Fischer's water content measurement device). Then, to the solution were added 663.5 mg (16.59 mmol) of 60% sodium hydride and 0.135 ml (7.49 mmol) of water while maintaining the liquid temperature to 9°C in an ice-bath and the mixture was stirred. The total water content in the system at this time was 0.65 equivalent relative to Compound 1. Then, 1.27 ml (13.4 mmol) of dimethyl sulfate was gradually added dropwise to the mixture to carry out an alkylation reaction at 10°C for 3 hours.

After completion of the alkylation reaction, 21 ml (23 mmol) of a saturated aqueous sodium hydrogen carbonate solution and 39 ml of methanol were added to the mixture to carry out a deprotection reaction under reflux (60 to 65°C) for 6 hours under nitrogen gas atmosphere. After completion of the deprotection reaction, the mixture was concentrated under reduced pressure, and 100 ml of ethyl acetate and 50 ml of water were added to the concentrate. The ethyl acetate layer was collected by separation, washed twice with each 50 ml of water and dried over anhydrous magnesium sulfate. After filtration, the filtrate was concentrated under reduced pressure, 16 ml of acetone and 13 ml of 10% aqueous ammonia were added to the concentrate, and the mixture was stirred at room temperature for one hour, and at 0 to 5°C for one hour to precipitate crystals. The obtained crystals were filtered and dried to give 6.23 g of 11-oxo-12-methoxy-8,9-anhydroerythromycin A 6,9-hemiketal (Compound 2) (Isolated yield based on Compound 1: 67%) as white crystals with a purity of 96% (analytical value by high performance liquid chromatography, analytical condition 2).

### Example 9

To a flask having an inner volume of 100 ml equipped with a stirring device, a thermometer, a reflux condenser and a dropping funnel were charged 10.0 g (12.76 mmol) of 2'-O-acetyl-4"-O-formyl-11-oxo-8,9-anhydroerythromycin A 6,9-hemiketal (Compound 1) and 90 ml of tetrahydrofuran under nitrogen atmosphere, the mixture was stirred at room temperature to make it uniform, and then, 50 ml of tetrahydrofuran was removed by distillation. When the water content in the system of this solution was measured, it was 350.8 ppm (Analytical value by Karl Fischer's water content measurement device). Then, to the solution was added 34 µl (1.89 mmol) of water at room temperature, the mixture was stirred for 20 minutes, and 664 mg (16.6 mmol) of 60% sodium hydride was added to the mixture while maintaining the liquid temperature to 9°C in an ice-bath and the mixture was stirred. The total water content in the system at this time was 0.22 equivalent relative to Compound 1. Then, 1.27 ml (13.4 mmol) of dimethyl sulfate was gradually added dropwise to the mixture to carry out an alkylation reaction at 5 to 10°C for 7 hours.

After completion of the alkylation reaction, 21 ml (23 mmol) of a saturated aqueous sodium hydrogen carbonate solution and 39 ml of methanol were added to the mixture to carry out a deprotection reaction under reflux (60 to 65°C) for 6 hours under nitrogen gas atmosphere. After completion of the deprotection reaction, the mixture was concentrated under reduced pressure, and 100 ml of ethyl acetate and 50 ml of water were added to the concentrate. The ethyl acetate layer was collected by separation, washed twice with each 50 ml of water and dried over anhydrous magnesium sulfate. After filtration, the mixture was concentrated under reduced pressure, 16 ml of acetone and 13 ml of 10% aqueous ammonia were added to the concentrate, and the resulting mixture was stirred at room temperature for one hour and at 0 to 5°C for one hour to precipitate crystals. The obtained crystals were filtered and dried to give 7.49 g of 11-oxo-12-methoxy-8,9-anhydroerythromycin A 6,9-hemiketal (Compound 2) (Isolated yield based on Compound 1: 80%) as white crystals with a purity of 95% (analytical value by high performance liquid chromatography, analytical condition 2).

### Example 10

To a flask having an inner volume of 50 ml equipped with a stirring device, a thermometer, a reflux condenser and a dropping funnel were charged 5.0 g (6.38 mmol) of 2'-O-acetyl-4"-O-formyl-11-oxo-8,9-anhydroerythromycin A 6,9-hemiketal (Compound 1) and 45 ml of tetrahydrofuran under nitrogen atmosphere, the mixture was stirred at room temperature to make it uniform, and then, 25 ml of tetrahydrofuran was removed by distillation. When the water content in the system of this solution was measured, it was 185 ppm (Analytical value by Karl Fischer's water content measurement device). Then, 31 µl (1.72 mmol) of water was added to the solution at room temperature and after stirring for 10 minutes, 332 mg (8.29 mmol) of 60% sodium hydride was added to the mixture while maintaining the liquid temperature to 5°C in an ice-bath and the mixture was stirred for 5 minutes. The total water content in the system at this time was 0.30 equivalent relative to Compound 1. Then, 0.6 ml (6.38 mmol) of dimethyl sulfate was gradually added dropwise to the mixture to carry out an alkylation reaction at 5 to 10°C for 5 hours.

After completion of the alkylation reaction, 11 ml (12 mmol) of a saturated aqueous sodium hydrogen carbonate solution and 19.5 ml of methanol were added to the mixture to carry out a deprotection reaction under reflux (60 to 65°C) for 9.5 hours under nitrogen gas atmosphere. After completion of the deprotection reaction, the mixture was concentrated under reduced pressure, and 50 ml of ethyl acetate and 25 ml of water were added to the concentrate. The ethyl acetate layer was collected by separation, washed twice with each 25 ml of water and dried over anhydrous magnesium sulfate. After filtration, the mixture was concentrated under reduced pressure, 8.1 ml of acetone and 6.3 ml of 10% aqueous ammonia were added to the concentrate, and the mixture was stirred at room temperature for one hour and at 0 to 5°C for one hour to precipitate crystals. The obtained crystals were filtered and dried to give 4.25 g of 11-oxo-12-methoxy-8,9-anhydroerythromycin A 6,9-hemiketal (Compound 2) (Isolated yield based on Compound 1: 92%) as white crystals with a purity of 95% (analytical value by high performance liquid chromatography, analytical condition 2).

### Example 11

To a flask having an inner volume of 100 ml equipped with a stirring device, a thermometer, a reflux condenser and a dropping funnel were charged 10.0 g (12.76 mmol) of 2'-O-acetyl-4"-O-formyl-11-oxo-8,9-anhydroerythromycin A 6,9-hemiketal (Compound 1) and 90 ml of tetrahydrofuran under nitrogen atmosphere, the mixture was stirred at room temperature to make it uniform, and then, 50 ml of tetrahydrofuran was removed by distillation. When the water content in the system of this solution was measured, it was 333.9 ppm (Analytical value by Karl Fischer's water content measurement device). Then, to the solution was added 52 µl (2.89 mmol) of water while maintaining the liquid temperature to 5°C in an ice-bath, and after stirring for 5 minutes, 740.5 mg (18.51 mmol) of 60% sodium hydride was added to the mixture and the resulting mixture was stirred for 5 minutes. The total water content in the system at this time was 0.43 equivalent relative to Compound 1. Then, 1.2 ml (12.96 mmol) of dimethyl sulfate was gradually added dropwise to the mixture to carry out an alkylation reaction at 5°C for 7 hours.

After completion of the alkylation reaction, 21 ml (23 mmol) of a saturated aqueous sodium hydrogen carbonate solution and 39 ml of methanol were added to the mixture, and a deprotection reaction was carried out under reflux (60 to 65°C) for 6 hours under nitrogen gas atmosphere. After completion of the deprotection reaction, the mixture was concentrated under reduced pressure, and 100 ml of ethyl acetate and 50 ml of water were added to the mixture. The ethyl acetate layer was collected by separation, washed twice with each 50 ml of water and dried over anhydrous magnesium sulfate. After filtration, the mixture was concentrated under reduced pressure, 16 ml of acetone and 13 ml of 10% aqueous ammonia were added to the concentrate, and the mixture was stirred at room temperature for one hour and at 0 to 5°C for one hour to precipitate crystals. The obtained crystals were filtered and dried to give 8.23 g of 11-oxo-12-methoxy-8,9-anhydroerythromycin A 6,9-hemiketal (Compound 2) (Isolated yield based on Compound 1: 92%) as white crystals with a purity of 94% (analytical value by high performance liquid chromatography, analytical condition 2).

### Example 12

To a flask having an inner volume of 100 ml equipped with a stirring device, a thermometer, a reflux condenser and a dropping funnel where charged 10.0 g (12.76 mmol) of 2'-O-acetyl-4"-O-formyl-11-oxo-8,9-anhydroerythromycin A 6,9-hemiketal (Compound 1) and 90 ml of tetrahydrofuran under nitrogen atmosphere, the mixture was stirred at room temperature to make it uniform, and then, 50 ml of tetrahydrofuran was removed by distillation. When the water content in the system of this solution was measured, it was 392 ppm (Analytical value by Karl Fischer's water content measurement device). Then, to the solution were added 52 µl (2.89 mmol) of water at room temperature and 664 mg (16.59 mmol) of 60% sodium hydride at 20°C, and after stirring the mixture, 30 µl (1.66 mmol) of water was added to the resulting mixture. The total water content in the system at this time was 0.43 equivalent relative to Compound 1. Then, 1.27 ml (13.4 mmol) of dimethyl sulfate was gradually addeddropwise to the mixture to carry out alkylation reaction at 20°C for 5 hours. Thereafter, 10.4 mg (0.26 mmol) of 60% sodium hydride and 0.025 ml (0.26 mmol) of dimethyl sulfate were additionally added to the mixture to carry out an alkylation reaction for one hour.

After completion of the alkylation reaction, 21 ml (23 mmol) of a saturated aqueous sodium hydrogen carbonate solution and 39 ml of methanol were added to the mixture to carry out a deprotection reaction under reflux (60 to 65°C) for 8 hours under nitrogen gas atmosphere. After completion of the deprotection reaction, the mixture was concentrated under reduced pressure, and 100 ml of ethyl acetate and 50 ml of water were added to the concentrate. The ethyl acetate layer was collected by separation, washed twice with each 50 ml of water and dried over anhydrous magnesium sulfate. After filtration, the mixture was concentrated under reduced pressure, 16 ml of acetone and 13 ml of 10% aqueous ammonia were added to the concentrate, and the resulting mixture was stirred at room temperature for one hour and at 0 to 5°C for one hour to precipitate crystals. The obtained crystals were filtered and dried to give 7.26 g of 11-oxo-12-methoxy-8,9-anhydroerythromycin A 6,9-hemiketal (Compound 2) (Isolated yield based on Compound 1: 78%) as white crystals with a purity of 96% (analytical value by high performance liquid chromatography, analytical condition 2).

### Example 13

To a flask having an inner volume of 100 ml equipped with a stirring device, a thermometer, a reflux condenser and a dropping funnel were charged 10.0 g (12.76 mmol) of 2'-O-acetyl-4"-O-formyl-11-oxo-8,9-anhydroerythromycin A 6,9-hemiketal (Compound 1) and 40 ml of tetrahydrofuran under nitrogen atmosphere, and the mixture was stirred at room temperature to make it uniform. When the water content in the system of this solution was measured, it was 2088.3 ppm (Analytical value by Karl Fischer's water content measurement device). Then, to the solution was added 664 mg (16.6 mmol) of 60% sodium hydride while maintaining the liquid temperature to 0°C in an ice-bath, and the mixture was stirred. The total water content in the system at this time was 0.41 equivalent relative to Compound 1. Then, 1.27 ml (13.4 mmol) of dimethyl sulfate was gradually added dropwise to the mixture to carry out alkylation reaction at 0°C for 6 hours. Thereafter, 10.4 mg (0.26 mmol) of 60% sodium hydride and 0.025 ml (0.26 mmol) of dimethyl sulfate were additionally added to the mixture to carry out an alkylation reaction for one hour.

After completion of the alkylation reaction, 21 ml (23 mmol) of a saturated aqueous sodium hydrogen carbonate solution and 39 ml of methanol were added to the mixture to carry out a deprotection reaction under reflux (60 to 65°C) for 8.5 hours under nitrogen gas atmosphere. After completion of the deprotection reaction, the mixture was concentrated under reduced pressure, and 100 ml of ethyl acetate and 50 ml of water were added. The ethyl acetate layer was collected by separation, washed twice with each 50 ml of water and dried over anhydrous magnesium sulfate. After filtration, the mixture was concentrated under reduced pressure, 16 ml of acetone and 13 ml of 10% aqueous ammonia were added to the concentrate, and the resulting mixture was stirred at room temperature for one hour and at 0 to 5°C for one hour to precipitate crystals. The obtained crystals were filtered and dried to give 7.55 g of 11-oxo-12-methoxy-8,9-anhydroerythromycin A 6,9-hemiketal (Compound 2) (Isolated yield based on Compound 1: 81%) as white crystals with a purity of 97% (analytical value by high performance liquid chromatography, analytical condition 2).

### Example 14

To a flask having an inner volume of 25 ml equipped with a stirring device, a thermometer, a reflux condenser and a dropping funnel were charged 2.50 g (3.19 mmol) of 2'-O-acetyl-4"-O-formyl-11-oxo-8,9-anhydroerythromycin A 6,9-hemiketal (Compound 1) and 20 ml of tetrahydrofuran under nitrogen atmosphere, the mixture was stirred at room temperature to make it uniform, and then, 10 ml of tetrahydrofuran was removed by distillation. When the water content in the system of this solution was measured, it was 492.18 ppm (Analytical value by Karl Fischer's water content measurement device). Then, 19.9 µl (1.08 mmol) of water was added to the solution at room temperature, and then, 166 mg (4.15 mmol) of 60% sodium hydride was added to the mixture while maintaining the liquid temperature to 5°C in an ice-bath and the resulting mixture was stirred. The total water content in the system at this time was 0.43 equivalent relative to Compound 1. Then, 0.51 ml (3.38 mmol) of methyl p-toluenesulfonate was gradually added dropwise to the mixture to carry out an alkylation reaction at 5 to 10°C for 20 hours. Thereafter, 12.8 mg (0.32 mmol) of 60% sodium hydride and 9.6 µl (0.063 mmol) of methyl p-toluenesulfonate were additionally added to the mixture to carry out an alkylation reaction for one hour.

After completion of the alkylation reaction, 5 ml (6 mmol) of a saturated aqueous sodium hydrogen carbonate solution and 9.8 ml of methanol were added to the mixture, and a deprotection reaction was carried out under reflux (60 to 65°C) for 5 hours under nitrogen gas atmosphere. After completion of the deprotection reaction, the mixture was concentrated under reduced pressure, and 25 ml of ethyl acetate and 12.5 ml of water were added to the concentrate. The ethyl acetate layer was collected by separation, washed twice with each 12.5 ml of water and dried over anhydrous magnesium sulfate. After filtration, the mixture was concentrated under reduced pressure, 4.1 ml of acetone and 3.1 ml of 10% aqueous ammonia were added to the concentrate, and the mixture was stirred at room temperature for one hour and at 0 to 5°C for one hour to precipitate crystals. The obtained crystals were filtered and dried to give 1.58 g of 11-oxo-12-methoxy-8,9-anhydroerythromycin A 6,9-hemiketal (Compound 2) (Isolated yield based on Compound 1: 69%) as white crystals with a purity of 96% (analytical value by high performance liquid chromatography, analytical condition 2).

### Example 15

To a flask having an inner volume of 200 ml equipped with a stirring device, a thermometer, a reflux condenser and a dropping funnel were charged 15.0 g (19.13 mmol) of 2'-O-acetyl-4"-O-formyl-11-oxo-8,9-anhydroerythromycin A 6,9-hemiketal (Compound 1) and 60 ml of tetrahydrofuran under nitrogen atmosphere, and the mixture was stirred at room temperature to make it uniform. When the water content in the system of this solution was measured, it was 660 ppm (Analytical value by Karl Fischer's water content measurement device). To the solution was added 18 µl (1.00 mmol) of water. The total water content in the system at this time was 0.18 equivalent relative to Compound 1. Then, 1063 mg (26.6 mmol) of 60% sodium hydride was added to the mixture while maintaining a liquid temperature to 5°C and the resulting mixture was stirred for 5 minutes. Then, 1.96 ml (20.73 mmol) of dimethyl sulfate was gradually added dropwise to the mixture to carry out an alkylation reaction at 5 to 10°C for 4 hours.

After completion of the alkylation reaction, 31 ml (35 mmol) of a saturated aqueous sodium hydrogen carbonate solution and 59 ml of methanol were added to the mixture to carry out a deprotection reaction under reflux (60 to 65°C) for 7 hours under nitrogen gas atmosphere. After completion of the deprotection reaction, the mixture was concentrated under reduced pressure, and 152 ml of ethyl acetate and 38 ml of water were added to the concentrate. The ethyl acetate layer was collected by separation, washed twice with each 76 ml of water and dried over anhydrous magnesium sulfate. After filtration, the mixture was concentrated under reduced pressure, 24 ml of acetone and 19 ml of 10% aqueous ammonia were added to the concentrate, and the resulting mixture was stirred at room temperature for one hour and at 0 to 5°C for one hour to precipitate crystals. The obtained crystals were filtered and dried to give 12.25 g of 11-oxo-12-methoxy-8,9-anhydroerythromycin A 6,9-hemiketal (Compound 2) (Isolated yield based on Compound 1: 88%) as white crystals with a purity of 98% (analytical value by high performance liquid chromatography, analytical condition 2).

### Example 16

To a flask having an inner volume of 200 ml equipped with a stirring device, a thermometer, a reflux condenser and a dropping funnel were charged 15.0 g (19.13 mmol) of 2'-O-acetyl-4"-O-formyl-11-oxo-8,9-anhydroerythromycin A 6,9-hemiketal (Compound 1) and 60 ml of tetrahydrofuran under nitrogen atmosphere, and the mixture was stirred at room temperature to make it uniform. When the water content in the system of this solution was measured, it was 1100 ppm (Analytical value by Karl Fischer's water content measurement device). The total water content in the system at this time was 0.22 equivalent relative to Compound 1. Then, 1065 mg (26.6 mmol) of 60% sodium hydride was added to the mixture while maintaining the liquid temperature to 5°C and the mixture was stirred for 5 minutes. Then, 1.96 ml (20.73 mmol) of dimethyl sulfate was gradually added dropwise to the mixture to carry out an alkylation reaction at 5 to 10°C for 3 hours.

After completion of the alkylation reaction, 31 ml (35 mmol) of a saturated aqueous sodium hydrogen carbonate solution and 59 ml of methanol were added to the mixture to carry out a deprotection reaction under reflux (60 to 65°C) for 7 hours under nitrogen gas atmosphere. After completion of the deprotection reaction, the mixture was concentrated under reduced pressure, and 152 ml of ethyl acetate and 38 ml of water were added to the concentrate. The ethyl acetate layer was collected by separation, washed twice with each 76 ml of water and dried over anhydrous magnesium sulfate. After filtration, the mixture was concentrated under reduced pressure, 24 ml of acetone and 19 ml of 10% aqueous ammonia were added to the concentrate, and the mixture was stirred at room temperature for one hour and at 0 to 5°C for one hour to precipitate crystals. The obtained crystals were filtered and dried to give 12.23 g of 11-oxo-12-methoxy-8,9-anhydroerythromycin A 6,9-hemiketal (Compound 2) (Isolated yield based on Compound 1: 88%) as white crystals with a purity of 98% (analytical value by high performance liquid chromatography, analytical condition 2).

### Comparative example 1

To a flask having an inner volume of 300 ml equipped with a stirring device, a thermometer, a reflux condenser and a dropping funnel were charged 11.0 g (14.0 mmol) of 2'-O-acetyl-4"-O-formyl-11-oxo-8,9-anhydroerythromycin A 6,9-hemiketal (Compound 1) and 44 ml of tetrahydrofuran under nitrogen atmosphere, and the mixture was stirred at room temperature to make it uniform. When the water content in the system of this solution was measured, it was 324.8 ppm (Analytical value by Karl Fischer's water content measurement device). To the solution was added 16.5 µl (0.92 mmol) of water. Then, 730 mg (18.2 mmol) of 60% sodium hydride was added to the mixture while maintaining the liquid temperature to 5°C in an ice-bath. The total water content in the system at this time was 0.13 equivalent relative to Compound 1. Then, 1.41 ml (14.9 mmol) of dimethyl sulfate was gradually added dropwise to the mixture at 5 to 10°C for 2.5 hours, and 11 µl (0.61 mmol) of water was additionally added to the mixture (The total water content in the system at this time was 0.17 equivalent relative to Compound 1.) to carry out an alkylation reaction for 4 hours. After completion of the alkylation reaction, when the reaction mixture was analyzed by high performance liquid chromatography (analytical condition 1), 2'-O-acetyl-4"-O-formyl-11-oxo-12-methoxy-8,9-anhydroerythromycin A 6,9-hemiketal (Compound 3) was formed merely about 20% or so, and thus, the alkylation reaction was stopped and a subsequent deprotection reaction was not carried out.

### Comparative example 2

To a flask having an inner volume of 330 ml equipped with a stirring device, a thermometer, a reflux condenser and a dropping funnel were charged 11.0 g (14.0 mmol) of 2'-O-acetyl-4"-O-formyl-11-oxo-8,9-anhydroerythromycin A 6,9-hemiketal (Compound 1) and 44 ml of tetrahydrofuran under nitrogen atmosphere, and the mixture was stirred at room temperature to make it uniform. When the water content in the system of this solution was measured, it was 1102 ppm (Analytical value by Karl Fischer's water content measurement device). To the solution was added 220 µl (12.2 mmol) of water. Then, 730 mg (18.2 mmol) of 60% sodium hydride was added to the mixture while maintaining the liquid temperature to 5°C in an ice-bath. The total water content in the system at this time was 1.09 equivalent relative to Compound 1. Then, 1.41 ml (14.9 mmol) of dimethyl sulfate was gradually added dropwise to the mixture to carry out an alkylation reaction at 5 to 10°C for 2.5 hours, and further, 56.1 mg (1.40 mmol) of 60% sodium hydride and 26.6 µl (0.28 mmol) of dimethyl sulfate were added to the same to carry out the reaction for 2 hours, and 56.1 mg (1.40 mmol) of 60% sodium hydride and 26.6 µl (0.28 mmol) of dimethyl sulfate were added to the same to carry out the reaction for 3 hours.

After completion of the alkylation reaction, 23 ml (25 mmol) of a saturated aqueous sodium hydrogen carbonate solution and 43 ml of methanol were added to the mixture to carry out a deprotection reaction under reflux (60 to 65°C) for 8.5 hours under nitrogen gas atmosphere. After completion of the deprotection reaction, the mixture was concentrated under reduced pressure, and 111 ml of ethyl acetate and 58 ml of water were added to the concentrate. The ethyl acetate layer was collected by separation, washed twice with each 50 ml of water and dried over anhydrous magnesium sulfate. After filtration, the mixture was concentrated under reduced pressure, 18 ml of acetone and 14 ml of 10% aqueous ammonia were added to the concentrate, and the mixture was stirred at room temperature for one hour and at 0 to 5°C for one hour to precipitate crystals. The obtained crystals were filtered and dried to give 6.1 g of 11-oxo-12-methoxy-8,9-anhydroerythromycin A 6,9-hemiketal (Compound 2) (Isolated yield based on Compound 1: 61%) as white crystals with a purity of 92% (analytical value by high performance liquid chromatography, analytical condition 2).

Incidentally, analytical conditions of high performance liquid chromatography in the above-mentioned Examples and Comparative examples are as follows.

### -Analytical condition 1-

Column: Kromasil KR100-5C18
Column temperature: 30°C
Eluent: Acetonitrile/water/28% aqueous ammonia (=700/300/3 (Volume ratio))
Flow rate: 1.0 ml/min.
Detection wavelength: 215 nm

### -Analytical condition 2-

Column: L-column ODS
Column temperature: 25°C
Eluent: Acetonitrile/water/28% aqueous ammonia (=725/275/40 (Volume ratio))
Flow rate: 1.0 ml/min.
Detection wavelength: 215 nm

### Utilizability in industry

According to the present invention, a 11-oxo-12-alkoxy-8,9-anhydroerythromycin A 6,9-hemiketal compound can be obtained with a high yield and high purity by a simple and easy method by alkylating 2'-O-acetyl-4"-O-formyl-11-oxo-8,9-anhydroerythromycin A 6,9-hemiketal compound with a high yield and high purity and then by deprotection.

## Claims

1. A process for preparing an erythromycin compound which comprises subjecting 2'-O-acetyl-4"-O-formyl-11-oxo-8,9-anhydroerythromycin A 6,9-hemiketal compound 1 represented by the formula (1) : 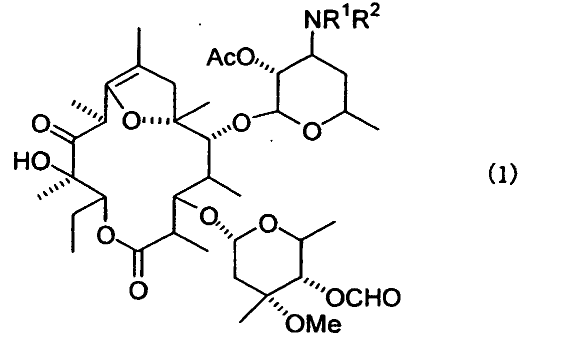 wherein R¹ and R² each independently represent a lower alkyl group,
to alkylation, wherein Compound 1 is reacted with an alkylating agent in the presence of a base in a mixed solvent of 0.18 to 1.05 equivalent of water based on the amount of Compound 1 and an organic solvent to give 2'-O-acetyl-4"-O-formyl-11-oxo-12-alkoxy-8,9-anhydroerythromycin A 6,9-hemiketal compound 3 represented by the formula (3) : 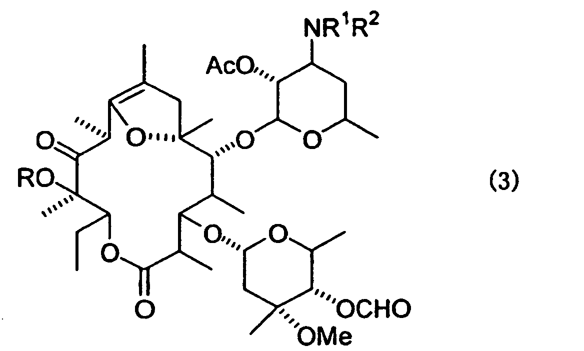 wherein R represents a lower alkyl group, and R¹ and
R² have the same meanings as defined above.

2. The preparation process according to Claim 1, wherein after alkylation, an acetyl group at the 2'-position and a formyl group at the 4"-position of Compound 3 are further removed to give 11-oxo-12-alkoxy-8,9-anhydroerythromycin A 6,9-hemiketal compound 2 represented by the formula (2): 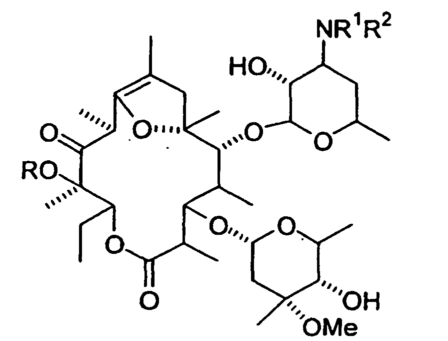 wherein R, R¹ and R² have the same meanings as defined in Claim 1.

3. The preparation process according to Claim 2, wherein alkylation and removal of the acetyl group and the formyl group of Compound 3 are carried out without isolating and purifying formed compound.

4. The preparation process according to any one of Claims 1 to 3, wherein R¹ and R² are each methyl group.

5. The preparation process according to any one of Claims 1 to 3, wherein R¹ is a methyl group, and R² is an isopropyl group.

6. The preparation process according to any one of Claims 1 to 5, wherein Compound 1 is reacted with the alkylating agent in a mixed solvent of water in an amount of 0.18 to 0.80 equivalent relative to the amount of Compound 1 and an organic solvent.

7. The preparation process according to any one of Claims 1 to 6, wherein Compound 1 is reacted with the alkylating agent in a mixed solvent of water in an amount of 0.18 to 0.70 equivalent relative to the amount of Compound 1 and an organic solvent.

8. The preparation process according to any one of Claims 1 to 7, wherein an organic solvent is an ether.

9. The preparation process according to any one of Claims 1 to 8, wherein the alkylating agent is at least one selected from the group consisting of dialkyl sulfate, alkyl alkylsulfonate and alkyl arylsulfonate.

10. The preparation process according to any one of Claims 1 to 8, wherein the alkylating agent is at least one selected from the group consisting of dialkylsulfate and alkyl arylsulfonate.

11. The preparation process according to any one of Claims 1 to 9, wherein the alkylating agent is at least one selected from the group consisting of dimethyl sulfate, diethyl sulfate, methyl methanesulfonate, methyl ethanesulfonate, ethyl methanesulfonate, methyl benzenesulfonate, methyl p-toluenesulfonate, p-bromomethyl benzenesulfonate and p-ethyl toluenesulfonate.

12. The preparation process according to any one of Claims 1 to 9, wherein the alkylating agent is dialkylsulfate selected from dimethyl sulfate and diethyl sulfate.

13. The preparation process according to any one of Claims 1 to 9, wherein the reaction is carried out by adding the alkylating agent after mixing the base, Compound 1 and the mixed solvent in the alkylation step.

## Patentansprüche

1. Verfahren zur Herstellung einer Erythromycinverbindung, das das Durchführen einer Alkylierung an der 2'-O-Acetyl-4"-O-formyl-11-oxo-8,9-anhydroerythromycin-A-6,9-hemiketalverbindung 1 der Formel (1): 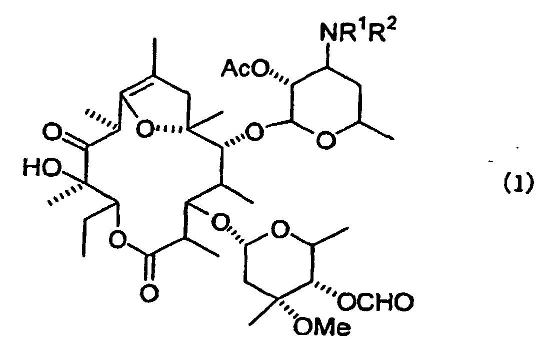 worin R¹ und R² jeweils unabhängig voneinander für eine Niederalkylgruppe stehen,
umfasst, wobei die Verbindung 1 mit einem Alkylierungsmittel in Gegenwart einer Base in einem Lösemittelgemisch aus 0,18 bis 1,05 Äquivalenten Wasser, bezogen auf die Menge der Verbindung 1, und einem organischen Lösemittel unter Bildung der 2'-O-Acetyl-4"-O-formyl-11-oxo-12-alkoxy-8,9-anhydroerythromycin-A-6,9-hemiketalverbindung 3 der Formel (3): 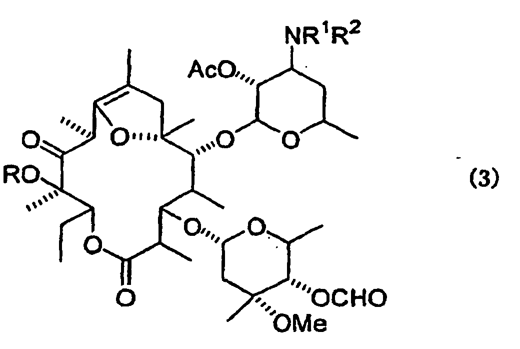 worin R für eine Niederalkylgruppe steht und R¹ und R² die oben angegebenen Bedeutungen haben,
umgesetzt wird.

2. Herstellungsverfahren nach Anspruch 1, wobei nach der Alkylierung eine Acetylgruppe an der 2'-Position und eine Formylgruppe an der 4"-Position von Verbindung 3 des weiteren entfernt werden, wobei die 11-Oxo-12-alkoxy-8,9-anhydroerythromycin-A-6,9-hemiketalverbindung 2 der Formel (2): 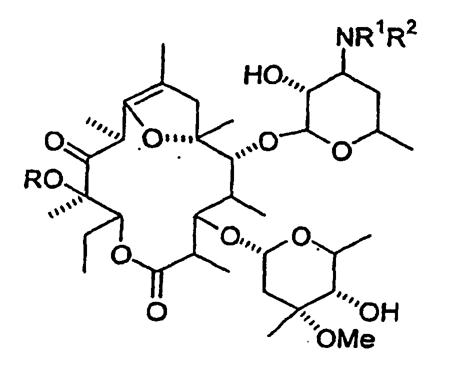 worin R, R¹ und R² die in Anspruch 1 definierten Bedeutungen haben,
erhalten wird.

3. Herstellungsverfahren nach Anspruch 2, wobei die Alkylierung und Entfernung der Acetylgruppe und der Formylgruppe von Verbindung 3 ohne Isolierung und Reinigung der gebildeten Verbindung durchgeführt werden.

4. Herstellungsverfahren nach einem der Ansprüche 1 bis 3, wobei R¹ und R² jeweils eine Methylgruppe sind.

5. Herstellungsverfahren nach einem der Ansprüche 1 bis 3, wobei R¹ eine Methylgruppe ist und R² eine Isopropylgruppe ist.

6. Herstellungsverfahren nach einem der Ansprüche 1 bis 5, wobei die Verbindung 1 mit dem Alkylierungsmittel in einem Lösemittelgemisch aus Wasser in einer Menge von 0,18 bis 0,80 Äquivalenten, bezogen auf die Menge der Verbindung 1, und einem organischen Lösemittel umgesetzt wird.

7. Herstellungsverfahren nach einem der Ansprüche 1 bis 6, wobei die Verbindung 1 mit dem Alkylierungsmittel in einem Lösemittelgemisch aus Wasser in einer Menge von 0,18 bis 0,70 Äquivalenten, bezogen auf die Menge der Verbindung 1, und einem organischen Lösemittel umgesetzt wird.

8. Herstellungsverfahren nach einem der Ansprüche 1 bis 7, wobei ein organisches Lösemittel ein Ether ist.

9. Herstellungsverfahren nach einem der Ansprüche 1 bis 8, wobei das Alkylierungsmittel mindestens ein Mittel ist, das aus der aus Dialkylsulfat, Alkylalkylsulfonat und Alkylarylsulfonat bestehenden Gruppe ausgewählt ist.

10. Herstellungsverfahren nach einem der Ansprüche 1 bis 8, wobei das Alkylierungsmittel mindestens ein Mittel ist, das aus der aus Dialkylsulfat und Alkylarylsulfonat bestehenden Gruppe ausgewählt ist.

11. Herstellungsverfahren nach einem der Ansprüche 1 bis 9, wobei das Alkylierungsmittel mindestens ein Mittel ist, das aus der aus Dimethylsulfat, Diethylsulfat, Methyl-methansulfonat, Methyl-ethansulfonat, Ethylmethansulfonat, Methyl-benzolsulfonat, Methyl-p-toluolsulfonat, p-Brommethyl-benzolsulfonat und p-Ethyl-toluolsulfonat bestehenden Gruppe ausgewählt ist.

12. Herstellungsverfahren nach einem der Ansprüche 1 bis 9, wobei das Alkylierungsmittel ein aus Dimethylsulfat und Diethylsulfat ausgewähltes Dialkylsulfat ist.

13. Herstellungsverfahren nach einem der Ansprüche 1 bis 9, wobei in der Alkylierungsstufe die Reaktion durch Zugabe des Alkylierungsmittels nach dem Mischen der Base, der Verbindung 1 und des Lösemittelgemischs durchgeführt wird.

## Revendications

1. Procédé pour préparer un composé d'érythromycine, qui comprend le fait de faire subir une alkylation à un composé 1 de 6,9-hemicétal de 2'-O-acétyl-4''-O-formyl-11-oxo-8,9-anhydroérythromycine A représenté par la formule (1): 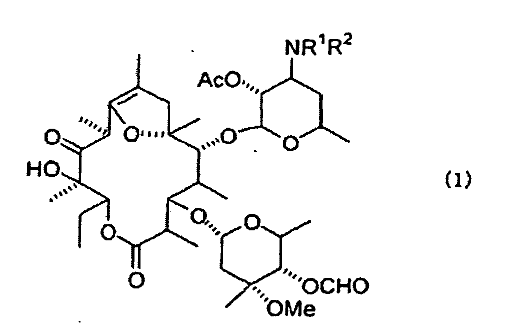 dans laquelle R¹ et R² représentent chacun indépendamment un groupe alkyle inférieur,
le composé 1 réagissant avec un agent d'alkylation en présence d'une base dans un solvant mixte qui contient de l'eau en quantité de 0,18 à 1,05 équivalent par rapport à la quantité du composé 1 et un solvant organique pour obtenir le composé 3 de 6,9-hemicétal de 2'-O-acétyl-4''-O-formyl-11-oxo-12-alcoxy-8,9-anhydroérythromycine A représenté par la formule (3): 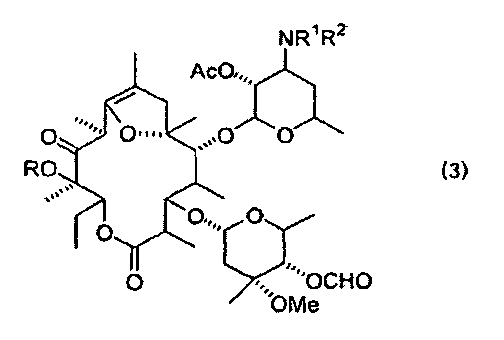 dans laquelle R représente un groupe alkyle inférieur, R¹ et R² ayant les mêmes significations que celles définies ci-dessus.

2. Procédé de préparation selon la revendication 1, dans lequel, après l'alkylation, un groupe acétyle en position 2' et un groupe formyle en position 4'' du composé 3 sont en outre extraits pour obtenir le composé 2 de 6,9-hémicétal de 11-oxo-12-alcoxy-8,9-anhydroérythromycine A représenté par la formule (2): 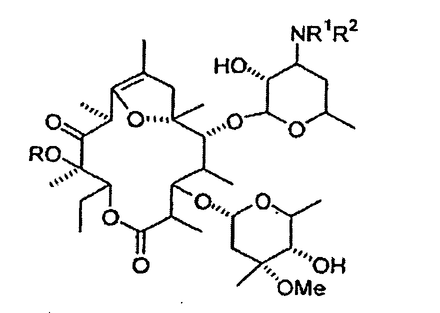 dans laquelle R, R¹ et R² ont les mêmes significations que celles définies en revendication 1.

3. Procédé de préparation selon la revendication 2, dans lequel l'alkylation et l'extraction du groupe acétyle et du groupe formyle du composé 3 sont réalisées sans isoler ni purifier le composé formé.

4. Procédé de préparation selon l'une quelconque des revendications 1 à 3, dans lequel R¹ et R² sont chacun un groupe méthyle.

5. Procédé de préparation selon l'une quelconque des revendications 1 à 3, dans lequel R¹ est un groupe méthyle, R² étant un groupe isopropyle.

6. Procédé de préparation selon l'une quelconque des revendications 1 à 5, dans lequel le composé 1 réagit avec l'agent d'alkylation dans un solvant mixte qui contient de l'eau en quantité de 0,18 à 0,80 équivalent par rapport à la quantité du composé 1 et un solvant organique.

7. Procédé de préparation selon l'une quelconque des revendications 1 à 6, dans lequel le composé 1 réagit avec l'agent d'alkylation dans un solvant mixte qui contient de l'eau en quantité de 0,18 à 0,70 équivalent par rapport à la quantité du composé 1 et un solvant organique.

8. Procédé de préparation selon l'une quelconque des revendications 1 à 7, dans lequel le solvant organique est un éther.

9. Procédé de préparation selon l'une quelconque des revendications 1 à 8, dans lequel l'agent d'alkylation est au moins un agent sélectionné dans l'ensemble constitué du sulfate de dialkyle, de l'alkylsulfonate d'alkyle et de l'arylsulfonate d'alkyle.

10. Procédé de préparation selon l'une quelconque des revendications 1 à 8, dans lequel l'agent d'alkylation est au moins un agent sélectionné dans l'ensemble constitué du sulfate de dialkyle et de l'arylsulfonate d'alkyle.

11. Procédé de préparation selon l'une quelconque des revendications 1 à 9, dans lequel l'agent d'alkylation est au moins un agent sélectionné dans l'ensemble constitué du sulfate de diméthyle, du sulfate de diéthyle, du méthanesulfonate de méthyle, de l'éthanesulfonate de méthyle, du méthanesulfonate d'éthyle, du benzènesulfonate de méthyle, du p-toluènesulfonate de méthyle, du benzènesulfonate de p-bromoéthyle et du toluènesulfonate de p-éthyle.

12. Procédé de préparation selon l'une quelconque des revendications 1 à 9, dans lequel l'agent d'alkylation est un sulfate de dialkyle sélectionné parmi le sulfate de diméthyle et le sulfate de diéthyle.

13. Procédé de préparation selon l'une quelconque des revendications 1 à 9, dans lequel la réaction est réalisée en ajoutant l'agent d'alkylation dans l'étape d'alkylation après avoir mélangé la base, le composé 1 et le solvant mixte.
